# EUROPEAN PATENT APPLICATION

(11) **EP 1 579 799 A1**
(43) Date of publication of application: **28.09.2005**
(21) Application number: 05004355.3
(22) Date of filing: 28.02.2005
(51) Int. Cl.: A61B 5/00

(54) **Apparatus and method for measuring quantitative value of information**

(30) Priority: 25.03.2004 JP 2004090330; 11.08.2004 JP 2004234718
(71) Applicant: Matsuoka, Sei, Nagasaki-ken (JP)
(72) Inventor: Matsuoka, Sei, Nagasaki-ken (JP)
(74) Representative: von Samson-Himmelstjerna, Friedrich

(57) **Abstract**

A measuring apparatus comprises a circular arc-shaped optical fiber, which is attached to a headband, and the two end sections of which point to touqiaoyin on the head, and an antenna coil that points to the forehead section, is worn on the head of a measurer. An O-ring test is used to evaluate the quantitative value of information of the information flowing through the optical fiber of the measuring apparatus when the measurer is facing a target object for measurement. When the O-ring test gives a value of <0> at the center of the optical fiber in the lengthwise direction, the quantitative value of information of the target object for measurement can be quantitatively determined from the measured value of the quantitative value of information flowing through the optical fiber at a position other than the center position.

## Description

### BACKGROUND OF THE INVENTION

### 1. Field of the Invention

The present invention relates to a technique for measuring the quantitative value of information that indicates the value of a biological tolerance relationship between a person, as the subject, and an object.

### 2. Description of the Related Art

One method used in medical diagnoses is a method called the O-ring test. The O-ring test is used to diagnose the location of abnormalities or the distribution of a specific substance within a body, as well as the suitability of a given treatment, based on the force required to open a ring formed of a thumb and another finger, which is typically the index finger (this will be referred to as O-ring). A diagnostic method using this O-ring test is described in detail in patent reference 1.

On the other hand, the inventor of the present invention has conceived of a system wherein when a subject grasps an object (any object, including substances), an interrelationship between the subject and the object is evident in terms of electromagnetic waves, and the biological relationship between the subject and the object is evaluated so that the information thereof (as employed herein, this information will be referred to as quantitative value information) is evaluated objectively, with consideration even given to interest tolerance. The system is referred to as a quantitative value information system. The inventor has studied the existence of such a quantitative value information system as well as the sending/receiving system of this information and have published the results of experiments (see non-patent reference 1, non-patent reference 2, non-patent reference 3, and patent reference 2). The O-ring test is one method that can be used for evaluating such quantitative value information. The O-ring advocated by the inventor is an O-ring formed by touching the tip of the index finger to the center of the fingerprint part of the thumb. In contrast, an O-ring formed by touching the tip of the thumb to the center of the fingerprint part of the index finger is known as a reverse O-ring. Experimental results that show the possibility of the existence of a quantitative value information system composed of the pineal body part of a human brain and the O-ring advocated by the inventor are disclosed in the non-patent reference 1. In the non-patent reference 2 and the patent reference 2, the inventor has verified the existence of this quantitative value information system, and have also disclosed a device which sends and receives this information using an energy generator. The non-patent reference 3 discloses contents relating to the information presented in the non-patent reference 1 and the non-patent reference 2.

As follows is a description of the concrete method of measuring a quantitative value of information disclosed in the non-patent reference 1. A red visible-light semiconductor laser device is placed on a desk. A 20 cc ampule of 20% glucose is placed 20 cm in front of the device and is irradiated with the laser, while an assistant puts the index fingers of both of his/her hands through an O-ring of an examiner, and attempts to pull open the O-ring with the right and left fingers, a total of two fingers. Here, if the O-ring remains closed, the evaluation is (+), or (+1) at this stage. Next, in order to open the O-ring of the examiner, the middle fingers of both hands of the assistant are added to increase the interlocking force when pulling apart laterally, and another attempt is made to pull the O-ring open with the right and left fingers, this time using a total of four fingers. This stage corresponds to (+2), and if the O-ring still does not open, then the assistant also puts the third fingers of both hands through the O-ring of the examiner, and again attempts to open the O-ring with the right and left fingers, now using a total of six fingers, which corresponds to (+3). If the O-ring opens with six fingers, then the range of the closure (+) is still (+2), and the single ampule of 20% glucose, which represents the object in this case, is evaluated to have a quantitative value of information of (+2), which corresponds to the stage before the O-ring opening. When a 20 cc ampule of 5% glucose is placed 20 cm in front of the laser device as an object and irradiated with the laser, the foregoing method results in a quantitative value of information of (+5).

If the O-ring opens at the first attempt, this indicates (-), and this (-) is then quantified by opening the O-ring using the above method, but with the hand which does not form the O-ring touching the rear end of the laser device under emission. The value resulting from pulling apart the O-ring under these conditions is the absolute value obtained by adding a (-) sign to the quantitative value of information of the object positioned in front of the laser device. Here, the polarities are symmetrical between the front and the rear, so that a (+) sign at the rear means a (-) sign for the object positioned in front, whereas the absolute values are the same both in front and behind the laser device. For example, with a 20 cc ampule of 50% glucose as the object, the foregoing method results in a quantitative value of information of (-4). Furthermore, with 5 cc of Oxyfull solution (H₂O₂) as the object, the foregoing method results in a quantitative value of information of (-6).

In addition, a description is also given for a method of measuring a quantitative value of information according to a physical equilibrium law across the laser device during laser emission. When a 20 cc ampule of 5% glucose (with a quantitative value of information of (+5) according to the foregoing results) and a 20 cc ampule of 50% glucose (with a quantitative value of information of (-4) according to the foregoing results) are stacked at a position in front of the laser device, and the quantitative value of information is measured, a result of (+1) is obtained. Similarly, if a 20 cc ampule of 5% glucose and a 20 cc ampule of 20% glucose (with a quantitative value of information of (+2)) are stacked, then the resulting quantitative value of information is (+7), Furthermore, if a 20 cc ampule of 5% glucose is placed in front of the laser device, an identical 20 cc ampule of 5% glucose is placed at the rear end of the laser device, and an O-ring test is then performed, the resulting value is <0> (zero). A value of <0> means a state where the first time the O-ring test is attempted the result is either a (+) or a (-), but the result of the next attempt is the opposite (-) or (+), and the third attempt is again the opposite (+) or (-), and so on, forever alternating between (-) and (+).

From the foregoing experimental results, it is inferred that the quantitative values of information obtained by O-ring tests using a laser device have polarities and display the addibility of quantified values. The use of this technique makes it possible to measure any value, no matter how high.

In addition, the non-patent reference 2 has proposed an apparatus for transmitting (feeding in) the quantitative value information of an object to another object. Namely, a red visible-light semiconductor laser device is used as a directional energy generator, and a sending object for feeding quantitative value information into a receiving object is put beside the light emitting part of the laser device. Then, the quantitative value of information of the sending object is fed into the receiving object, a piece of white paper placed at a distance of 10 cm. After 10 minutes, if the sending object is put at the rear end of the laser device, and the piece of white paper, into which the quantitative value of information was fed, is irradiated with the laser device, and measurement is then conducted using the O-ring test, the result is a <0> value. This result indicates that the piece of white paper has the same quantitative value of information as that of the sending object fed in, or in other words, that the quantitative value of information of the sending object was projected and left in the piece of white paper, the receiving object. The same results were also observed after a lapse of 24 hours and a lapse of one week, with no variation (attenuation) of the quantitative value of information within these periods.

Based on the foregoing verifications, in order to enable the transmission of a more complicated macromolecular biological object in the form of information from the quantitative value of information system, the inventor has also proposed a transmitting apparatus which achieves a three-dimensional, higher integration of the quantitative value of information. This apparatus comprises two red visible-light semiconductor laser devices (A, B) having side inputs for accepting different pieces of information, and a laser demodulator (a light receiving and transferring device). The laser demodulator converts an electrical signal input into an optical signal, and performs a demodulating action which converts an optical signal into an electrical signal, and then returns the electrical signal into an optical signal.

Using this apparatus, the sending object was placed on the near side of the side input of the laser device (A), and a piece of white paper, which functioned as the receiving object and was placed 10 cm in front of the laser device (B), was irradiated through the laser demodulator for 20 seconds so that the quantitative value information of the sending object was fed into the receiving object. When the quantitative value of information at a narrow area where the central light spot was fed onto this white paper was measured, it was confirmed that the original value of the quantitative value of information had been integrated when the quantitative value information was transmitted, thus amplifying the quantitative value of information.

In addition, in the patent reference 2, the inventor has proposed a quantitative value information transmitting apparatus which improves the energy generating device described in the non-patent reference 2, making it possible to transmit beneficial quantitative value information with higher efficiency. This quantitative value information transmitting apparatus is an apparatus for transmitting the quantitative value of information of a sending object to a receiving object using a directional energy generator, wherein the energy generator comprises a combination of a magnetism generator and an ion generator. According to this energy generator, the magnetism generator and the ion generator produce a synergistic effect which provides a quantitative value of information that has been integrated to between several thousand times and several tens of thousands of times that of a quantitative value information transmitting apparatus using the laser device described in the non-patent reference 2. The quantitative value of information that has been integrated and amplified to a high value and transmitted by this energy generator is then stored in a receiving spot, stably and without attenuation, for a predetermined period or beyond.

On the other hand, when making a diagnosis using the O-ring test described in the patent reference 1, several points of difficulty arise, including the fact that at least two people are required, the examiner and the examinee, and the fact that because the diagnostic method is based completely on manual strength, a considerable burden is placed on the examiner. Furthermore, the physical measurement of the level of force required to open the O-ring is both unsatisfactory and inaccurate. This means the diagnosis must rely on the experience of the examiner, which in turn means an experienced examiner is required. In order to solve these problems, a variety of apparatuses have been proposed for objectively and quantitatively measuring either the level of force required to open the O-ring, or the muscular force of the fingers (see patent references 3 through 5, for example).
Patent reference 1: U.S. Pat. No. 5,188,107
   Non-patent reference 1: *Jintai Kagaku [Mind-Body Science]* (issued by the Society for Mind-Body Science), 1999, Vol. 8, No. 2, pp. 57-71
   Non-patent reference 2: *Jintai Kagaku [Mind-Body Science]* (issued by the Society for Mind-Body Science), 2000, Vol. 9, No. 2, pp. 41-56
   Non-patent reference 3: "Quantitative Value of Information", [online], Hakusei Matsuoka, identified 22nd November 2004, URL: http://www.hmatuoka.com/index.html
Patent reference 2: Unexamined Japanese Patent Publication No. 2003-50300
Patent reference 3: Japanese Patent Publication No. Hei 3-44525
Patent reference 4: Japanese Patent No. 2,678,334
Patent reference 5: Japanese Patent No. 3,456,612

The measuring apparatuses disclosed in the patent references 3 through 5 above offer certain advantages in that the examinee, that is the patient, can objectively and quantitatively measure the muscular force of the O-ring by him or herself, without requiring the presence of an examiner or other third parties. However, such measuring apparatuses are not suitable for measuring quantitative values of information in the quantitative value information system advocated by the inventor of the present invention. This is because the measuring apparatuses disclosed in the patent references 3 through 5 are premised upon the O-ring tests being performed in the context of medical diagnoses, as described in the patent reference 1. Furthermore, they cannot be used in a system where energy vibrations intrinsic to an object (substance) are received by the pineal body part of the brain of a subject (human), and are evaluated in terms of a biological tolerance relationship between the object and the subject in the pineal body part, as a value with a polarity. Particularly, in the case of objects whose quantitative value of information has been enhanced to a value higher than the original quantitative value of information of the object through the use of a transmission apparatus as described in the patent reference 2, differences in the mechanics of muscle physiology relating to the O-ring test mean that it is in fact impossible to measure such high quantitative values of information using the measuring apparatuses described in the patent references 3 through 5.

Furthermore, the method of measuring quantitative values of information disclosed by the inventor in the non-patent reference 1 presents a problem in that the method requires a special piece of precision mechanical equipment called a red visible-light semiconductor laser device, which is difficult for an ordinary person to operate, and this limits the number of people who can perform the measurement. Furthermore, the number of times the O-ring is tested increases with higher quantitative values of information, and this not only increases the complexity, but also places a greater burden on the assistant conducting the O-ring tests. In addition, the higher the quantitative value of information of the object, the greater the quantity of measuring auxiliary objects of known quantitative value of information that must be placed at the rear of the laser device before the evaluated value reaches <0> zero, which also increases the complexity.

### SUMMARY OF THE INVENTION

An object of the present invention is to provide a measuring apparatus and a measuring method which enable the measurement of quantitative values of information objectively and quantitatively, within a quantitative value information system which evaluates the biological tolerance relationship between a person, who represents the subject, and an object, using an apparatus with a simple construction.

The inventor of the present invention noticed that in the transmission path for quantitative value information within the human body, centered about the pineal body part of the human brain, the reactive sites which resonate with the pineal body part exist within a path which encircles the frontal, temporal, and occipital regions, and discovered that by sending and receiving information between the inside and outside of the human brain through a specific group of these reactive sites, it is possible to measure the quantitative values of information evaluated in the pineal body part, and they were thus able to complete the present invention.

In other words, a measuring apparatus according to the present invention is used for measuring quantitative values of information in a quantitative value information system which evaluates a biological tolerance relationship between the pineal body part of the brain of a person, who represents the subject, and an object, and comprises a hat body capable of being worn on the head of the person; a cord attached to the hat body, with end sections that point to the right and left touqiaoyin, respectively, which are meridian points in the rear temporal region, and which forms a circular arc shape to the rear of the head in its entirety; and a metal antenna coil, which is attached to the hat body and points to the forehead of the person. Here, either an optical fiber or a conductive wire can be used as the cord. Furthermore, metal auxiliary coils can also be attached to the respective ends of the cord.

Furthermore, a measuring method according to the present invention is used to measure quantitative values of information in a quantitative value information system which evaluates a biological tolerance relationship between the pineal body part of the brain of a person, who represents the subject, and an object. In this method, a measurer places the aforementioned measuring apparatus on his or her head so that the end sections of the cord point to the respective touqiaoyin points in the rear temporal region, and uses the O-ring test to determine the quantitative value of information for the information flowing through the cord of the measuring apparatus when the measurer faces the object for measurement. Then, when the value for the O-ring test at the center of the cord in a lengthwise direction is zero <0>, the quantitative value of information of the object for measurement is determined from the value of the quantitative value of information flowing through the cord at a position other than the center of the cord. The method of determining the quantitative value of information of the object for measurement may involve: attaching an auxiliary object with a known quantitative value of information to the cord at a position other than the aforementioned center position, or providing an auxiliary object with a known quantitative value of information in iterative contact with the cord at a position other than the center position; and then determining the quantitative value of information of the object for measurement from the determined value of the quantitative value of information flowing through the cord, except at the center of the cord, when the value of the O-ring test at the center position of the cord is zero <0>.

When the person who represents the subject places the measuring apparatus according to the present invention on his or her head and faces the target object for measurement, the quantitative value information of the target object for measurement is sent, in the form of electromagnetic waves, to the pineal body part of the person via the antenna coil positioned at his or her forehead. Then, the value of the biological tolerance relationship between the person and the target object for measurement, that is the quantitative value of information, is evaluated in the pineal body part. The quantitative value information generated in the pineal body part flows from the pineal body part towards the touqiaoyin on the left side of the head, from where it is transmitted, in the form of electromagnetic waves, to the end of the cord pointing to the left side touqiaoyin. The quantitative value information then flows through the inside of the cord, is transmitted, in the form of electromagnetic waves, from the end of the cord pointing to the right side touqiaoyin to the right side touqiaoyin, and then returns to the pineal body part. At this time, if an auxiliary coil is attached to each end of the cord, the ability to transmit the quantitative value information between the touqiaoyin and the ends of the cord is enhanced.

By simply wearing the measuring apparatus of the present invention on the head, an information transmission circuit is formed from the pineal body part, to the left side touqiaoyin, the cord, the right side touqiaoyin, and then back to the pineal body part. Because the quantitative value information of the target object for measurement generated in the pineal body part flows through this information transmission circuit, which passes through the pineal body part and the cord of the measuring apparatus, the quantitative value of information of the target object for measurement can be determined by evaluating the quantitative value information flowing through this information transmission circuit. Accordingly, the quantitative value of information of the target object for measurement can be measured objectively and quantitatively, with a considerably reduced burden on the assistant performing the O-ring test, without using any special precision mechanical equipment, but rather, using an apparatus with a simple construction.

### BRIEF DESCRIPTION OF THE DRAWINGS

Fig. 1 is a perspective view showing the basic construction of a quantitative value of information measuring apparatus used in examples of the present invention;
Fig. 2 is a diagram showing the measuring apparatus of Fig. 1 being worn on the head;
Fig. 3 is a schematic diagram showing the flow of quantitative value information when the measuring apparatus in Fig. 1 is worn on the head;
Fig. 4 is a diagram showing a state where auxiliary objects are attached to the measuring apparatus; and
Fig. 5 is a diagram showing the pathways and meridian points of the head.

### DETAILED DESCRIPTION OF THE PREFERRED EMBODIMENTS

The measuring apparatus of the present invention is an apparatus for measuring quantitative values of information in the quantitative value information system advocated by the inventor of the present invention, and differs fundamentally from the apparatuses for measuring the level of force required to open an O-ring or the muscular force of the fingers as described in the patent references 3 through 5, which are premised upon the O-ring test being performed in the context of a medical diagnosis. The quantitative value of information in the quantitative value information system indicates the value of the biological tolerance relationship between a person, who represents the subject, and a target object for measurement. Here, the target object for measurement can be anything (any object, including any type of substance) which affects the subject directly or indirectly in his or her life, and is not restricted to objects related to medicine. Furthermore, the quantitative value of information of the target object for measurement includes the inherent quantitative value of information of the target object (or an additive value in the case of multiple objects), as well as quantitative values of information which have been integrated to generate a higher value, using a quantitative value information transmitting apparatus. The quantitative value of information is evaluated as a non-contiguous characteristic integer, and a polarity (+ or -).

The measuring apparatus of the present invention is used while worn on the head of a person. For this reason, a cord which functions as an input and output terminal for transmitting the quantitative value information between the apparatus and the pineal body section, and an antenna coil for efficiently transmitting the quantitative value information of the target object for measurement to the pineal body section, are attached to a hat body. The resulted hat body can be worn on the head. There are no particular limitations on the shape of the hat body, or on the material from which it is made, provided that both ends of the cord can be attached at positions which point to the right and left touqiaoyin, respectively, which are meridian points in the human occipital region, and the antenna coil can be attached at a position which points to the forehead region of the person. As such, a belt-like or hat-like shape is suitable for the shape of the hat body, and suitable materials include woven and unwoven fabrics, leather, and plastic. In practical terms, the most suitable hat body is a band-shaped body made of plastic.

The antenna coil improves and stabilizes the transmission efficiency when the quantitative value information of the target object for measurement is transmitted to the pineal body part of a person in the form of electromagnetic waves. The quantitative value information of the target object for measurement is transmitted to the pineal body part of the person via this antenna coil, and the value of the biological tolerance relationship between the person and the target object, that is the quantitative value of information, is evaluated in the pineal body part. If this antenna coil is made of a metal, the transmission efficiency can be effectively improved and stabilized regardless of the size and shape of the antenna coil, although a coil made of an iron alloy with an outside diameter of approximately 25 to 30 mm, which is wound anticlockwise from the outward direction towards the hat body, is preferred.

The cord is attached to the hat body at such a position that the ends of the cord point to the right and left touqiaoyin, respectively, which are meridian points in the occipital region. The touqiaoyin are one of the meridian points of the human body in Oriental medicine, and lie on the small intestine and gall bladder meridians. What is remarkable about touqiaoyin is that the polarity and information values are (-6) for the quantitative value of information of the left touqiaoyin, and (+6) for the quantitative value of information of the right touqiaoyin. This suggests that there is movement of information between the right and left touqiaoyin. Suitable materials for making the cord include optical fibers or conductive wires such as copper or aluminum, which allow favorable transmission of photons or electrons. Although the thickness of the cord has no direct relationship to the ability of the cord to transmit value information, a thickness which allows the cord to be attached easily to the hat body and which does not prove an encumbrance when the measuring apparatus is placed on the head is preferred. For example, an outside diameter of approximately 2 to 5 mm is ideal. The overall shape of the cord is a circular arc shape formed at the rear of the head. In addition, an auxiliary coil made of metal is preferably attached to each end of the cord.

Because the ends of the cord point to the right and left touqiaoyin, respectively, the quantitative value information generated in the pineal body part flows from the pineal body part towards the left side touqiaoyin, is transmitted in the form of electromagnetic waves to the end of the cord pointing to the left side touqiaoyin, flows through the cord, and is then transmitted in the form of electromagnetic waves from the end of the cord pointing to the right side touqiaoyin into the right side touqiaoyin, before returning to the pineal body part. At this time, if auxiliary coils that are wound anticlockwise relative to a direction from the left side end of the cord to the right side end are attached to the ends of the cord, then the ability to transmit quantitative value information between the touqiaoyin and the ends of the cord is enhanced and stabilized. The directionality of the information transmission circuit from the pineal body part, to the left side touqiaoyin, the cord, the right side touqiaoyin, and then back to the pineal body part, is also improved. If these auxiliary coils are made of metal, the transmission capability can be improved regardless of the size and shape of the auxiliary coils, although coils made of iron alloy with an outside diameter of approximately 25 to 30 mm are preferred.

The method of measuring quantitative values of information using the measuring apparatus described above is implemented as follows. First, the measurer places the measuring apparatus on his or her head. The information that flows through the cord while there is no target object for measurement in front of the measurer is then evaluated using the O-ring test. The information that flows through the cord in the absence of a target object is the information sensed unconsciously by the pineal body part of the measurer wearing the measuring apparatus, and does not indicate the value of a biological tolerance relationship between the measurer and the measuring apparatus. In the prototype measuring apparatus produced by the inventor of the present invention, information with an absolute value of (2) flows through the cord when the measurer places the measuring apparatus on his or her head.

Next, the quantitative value information that flows through the cord when the measurer who is wearing the measuring apparatus on his or her head is facing a target object for measurement is evaluated using the O-ring test. At this time, the quantitative value of information of the target object for measurement is evaluated in the pineal body part of the measurer, and the resulting quantitative value information then flows through the cord. The quantitative value information of the target object for measurement is transmitted, in the form of electromagnetic waves, via the antenna coil of the measuring apparatus, to the pineal body part of the measurer, and the value of the biological tolerance relationship between the measurer and the target object for measurement, that is the quantitative value of information, is evaluated in the pineal body part. The quantitative value information generated in the pineal body part then flows from the pineal body part towards the left side touqiaoyin, is transmitted in the form of electromagnetic waves to the end of the cord pointing to the left side touqiaoyin, flows through the inside of the cord, and is then transmitted from the cord end section on the opposite side, in the form of electromagnetic waves, to the right side touqiaoyin, from where it returns to the pineal body part. At this time, the auxiliary coils attached to the ends of the cord fulfill the function of enhancing and stabilizing the ability to transmit quantitative value information between the right and left touqiaoyin and the ends of the cord.

The inventor of the present invention has confirmed through experimentation that when this measuring apparatus is used, the quantitative value information which flows through the information transmission circuit from the pineal body part, to the left side touqiaoyin, the cord, the right side touqiaoyin, and then back to the pineal body part, has a (-) polarity from the pineal body part to the center of the cord, a (+) polarity in the return path from the center of the cord to the pineal body part, and has a value of <0> at the center position of the cord, and that the quantitative value of information at the position of the cord end section in the return path corresponds to the quantitative value of information of the object. Accordingly, when the O-ring test is used to evaluate the quantitative value information flowing through the cord while the measurer faces a target object for measurement, if it can be confirmed that the value at the center position of the cord is <0>, then the absolute value of the result of evaluating the quantitative value information flowing in the cord at that time using the O-ring test will correspond to the absolute value of the quantitative value of information of the target object for measurement. The reason that a value of <0> is measured at the center position of the cord is that this point resonates electromagnetically with the pineal body part.

Therefore, to measure the quantitative value of information of the target object for measurement, first an O-ring test is performed to confirm that the value at the center position of the cord is <0> when the measurer wearing the measuring apparatus on his or her head faces the target object, and next, the O-ring test is performed a second time to find out whether the polarity in this situation is (-) or (+). If the O-ring test shows that the polarity under these conditions, that is the polarity of the target object for measurement, is (+), then the measuring operation is performed in the (+) polarity return path of the cord, whereas if the polarity is shown to be (-), the measuring operation is performed in the (-) polarity outgoing path of the cord. If the quantitative value of information of the target object is high, then in order to reduce the burden on the assistant performing the O-ring test, auxiliary objects comprising objects with known quantitative values of information can be attached to either the return path or the outgoing path of the cord according to the polarity in this situation, and this attachment and addition of auxiliary objects are continued until the O-ring test shows a value of <0> at the center position of the cord. The sum of the quantitative values of information of the auxiliary objects when a value of <0> is obtained at the center position of the cord is the quantitative value of information of the target object for measurement. Another method is to provide an auxiliary object with a known quantitative value of information in iterative contact with either the return path or the outgoing path of the cord, and count the number of contact iterations required until the O-ring test shows a value of <0> at the center position of the cord. The number of contact iterations required before the O-ring test shows a value of <0> at the center position of the cord is the same as the absolute value of the quantitative value of information of the target object for measurement.

### EXAMPLES

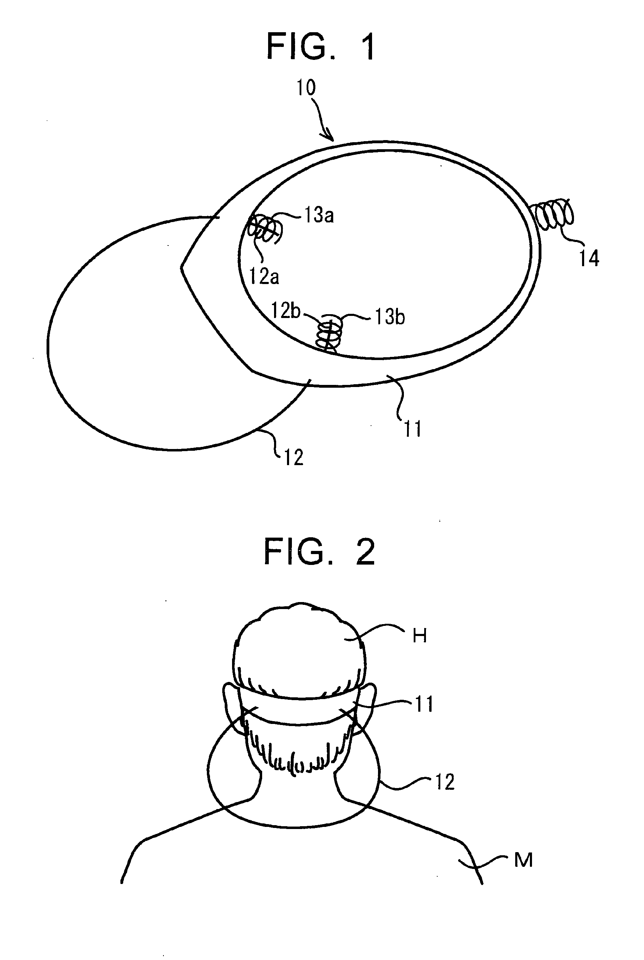

Fig. 1 is a perspective view showing the basic structure of a quantitative value information measuring apparatus used in examples of the present invention. Fig. 2 is a diagram showing the measuring apparatus of Fig. 1 being worn on the head.

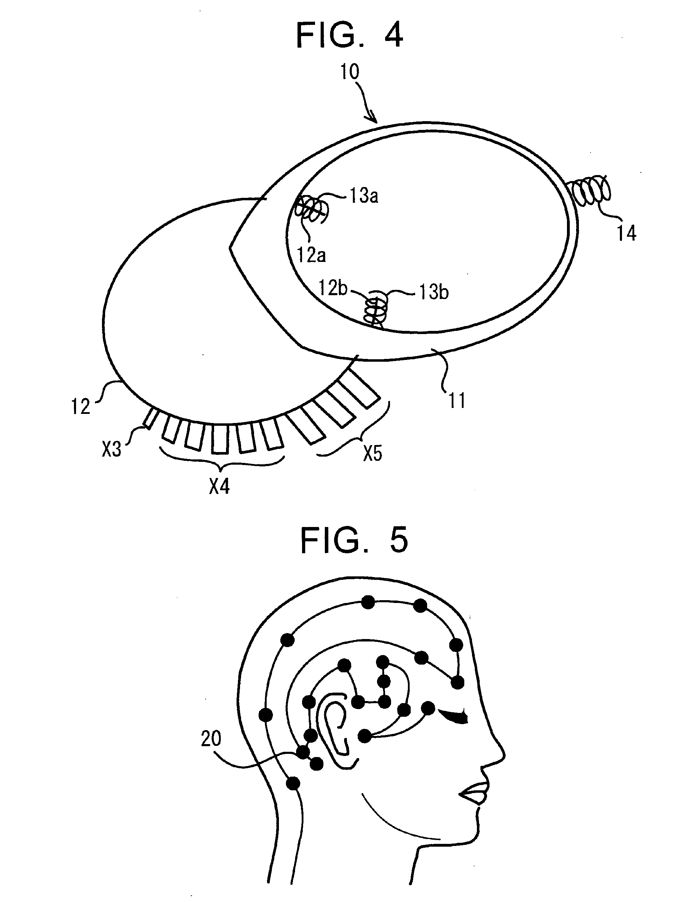

The basic structure of the measuring apparatus 10 of this embodiment comprises a band-shaped hat body (hereafter referred to as a headband) 11, a cord (hereafter referred to as an optical fiber) 12 which is made of optical fiber, auxiliary coils 13a and 13b made of iron alloy, and an antenna coil 14 made of iron alloy. The headband 11 is capable of being worn on a head H of a measurer M. The optical fiber 12 is attached to the headband 11, and two end sections 12a and 12b thereof point to the right and left touqiaoyin, respectively, which are meridian points in the rear temporal region (see reference numeral 20 in Fig. 5, which shows the pathways and meridian points of the head). The auxiliary coils 13a and 13b are attached to the end sections 12a and 12b. The antenna coil 14 is attached to the headband 11 and points to the forehead region of the measurer M (not shown in the drawings).

The headband 11 is a band made of a synthetic resin, of a size that prevents the band from slipping down when worn on the head H. The end sections 12a and 12b of the optical fiber 12 are attached to the headband 11 at positions that correspond to the right and left touqiaoyin in the rear temporal region when the headband 11 is worn. The optical fiber 12 is a circular-arc-shaped single fiber with an outside diameter of approximately 3 mm and a length of approximately 60 cm. The end sections 12a and 12b of the optical fiber 12 are attached so as to point to the right and left touqiaoyin, respectively.

The auxiliary coils 13a and 13b are attached to the end sections 12a and 12b of the optical fiber 12. The auxiliary coil 13a is a helical coil that is wound anticlockwise from the tip end of the end section 12a towards the base end, and the auxiliary coil 13b is a helical coil that is wound anticlockwise from the base end of the end section 12b to the tip end. In addition, the antenna coil 14 is attached at a position that corresponds to the forehead when the headband 11 is worn. The antenna coil 14 is a helical coil that is wound anticlockwise from the outside towards the headband 11.

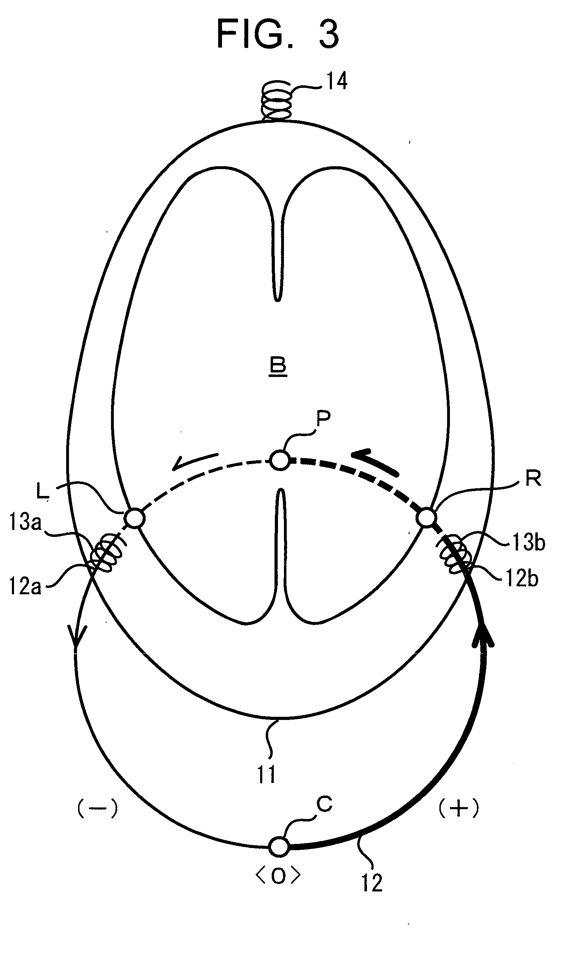

Fig. 3 is a schematic diagram showing the flow of quantitative value information when the measuring apparatus 10 is worn on the head H. In this diagram, the symbol B indicates the brain of the measurer M, P indicates the pineal body part, L indicates the left side touqiaoyin, and R indicates the right side touqiaoyin. When the measurer M wears the measuring apparatus 10 on his or her head H, and faces a target object for measurement (not shown in the figure), the quantitative value information of the target object is transmitted, in the form of electromagnetic waves, to the pineal body part P of the brain B via the antenna coil 14 of the measuring apparatus 10. The value of the biological tolerance relationship between the measurer M and the object, that is the quantitative value of information, is evaluated in the pineal body part P. The quantitative value information generated in the pineal body part P then flows from the pineal body part P towards the left side touqiaoyin L, is transmitted in the form of electromagnetic waves to the end section 12a of the optical fiber pointing toward the left side touqiaoyin L, flows through the inside of the optical fiber 12, and is then transmitted in the form of electromagnetic waves from the end section 12b of the optical fiber to the right side touqiaoyin R, before returning to the pineal body part P. Then, the auxiliary coils 13a and 13b attached to the end sections 12a and 12b of the optical fiber perform the function of enhancing and stabilizing the ability to transmit quantitative value information between the right and left touqiaoyin R and L and the end sections 12a and 12b of the optical fiber. It is thought that when the measuring apparatus 10 is worn on the head H, if there is either no target object for measurement in front of the measurer, or the measurer M is indifferent to the object, then an information value of (+2) is evaluated within the pineal body part P, and information with an absolute value of (2) flows through the optical fiber 12, but if the measurer then notices and takes interest in the target object for measurement, the activity for evaluating quantitative value information in the pineal body part P switches from an indifferent state to an evaluation-active state, and the quantitative value information of the target object for measurement flows through the information transmission circuit.

As follows is a description of a method of measuring quantitative values of information using the measuring apparatus 10, based on specific examples. As described in the non-patent reference 1, it is known that when a 20 cc ampule of 5% glucose is placed in front of a laser device, as the target object for measurement, and is irradiated with the laser while the quantitative value of information of the substance is measured using the O-ring test, then the quantitative value of information for the 5% glucose ampule is (+5). The following description assumes the use of a 20 cc ampule of 5% glucose as the target object for measurement, with the quantitative value of information of this 20 cc ampule of 5% glucose being measured by a measurer M wearing the measuring apparatus 10 on his or her head and facing the 20 cc 5% glucose ampule at a distance of 2 m.

When the measurer M wearing the measuring apparatus 10 on his or her head faces the target object for measurement, as described above, the quantitative value information generated in the pineal body part P of the measurer M flows through an information transmission circuit from the pineal body part P, to the left side touqiaoyin L, the optical fiber end section 12a, the optical fiber end section 12b, the right side touqiaoyin R, and then back to the pineal body part P. In this case, the target object for measurement is a 20 cc ampule of 5% glucose, and consequently the quantitative value of information is evaluated as (+5) by the pineal body part P, and this quantitative value information should be flowing through the above information transmission circuit. In order to confirm this, a test was performed which involved opening the O-ring of the measurer M, while irradiating a laser onto the optical fiber 12, in the same manner as in the measurement method described in the non-patent reference 1. The results showed that from the position of the optical fiber end section 12a to a position at the center C of the optical fiber, the quantitative value of information was (-5); and from the position at the center C of the optical fiber to the optical fiber end section 12b, the quantitative value of information was (+5), whereas at the center C of the optical fiber the value was <0>. These experimental results confirmed that the quantitative value information flowing in the aforementioned information transmission circuit had a (-) polarity in the portion from the pineal body part P through the left side touqiaoyin L and the optical fiber end section 12a to the center C of the optical fiber, and a (+) polarity in the portion from the center C of the optical fiber through the optical fiber end section 12b and the right side touqiaoyin R to the pineal body part P, that the value was <0> at the center C of the optical fiber, and that the quantitative value of information at the position of the optical fiber end section 12b corresponded with the quantitative value of information of the target object for measurement. In other words, this means that when an O-ring test confirmed a <0> value at the center C of the optical fiber, which represents the point that resonates electromagnetically with the pineal body part P, the value of the quantitative value information flowing through the optical fiber 12, as evaluated by the O-ring test, corresponded to the quantitative value of information of the target object for measurement. If polarity is also considered, then the quantitative value of information at the position of the optical fiber end section 12b corresponded to the quantitative value of information of the target object.

The following is a summary of the method of measuring quantitative values of information using the measuring apparatus 10. When the measurer M who is wearing the measuring apparatus 10 on his or her head faces a target object for measurement, the quantitative value information of the target object is transmitted to the pineal body part P of the measurer M via the antenna coil 14 at the forehead of the measurer M, and this quantitative value of information is then evaluated. The quantitative value information generated by the pineal body part P flows from the optical fiber end section 12a toward the optical fiber end section 12b. At this time, if the O-ring test confirms that the value at the center position C of the optical fiber is <0>, then the quantitative value of information at the optical fiber end section 12b at that time corresponds to the quantitative value of information of the target object for measurement. In other words, the quantitative value of information of the target object for measurement can be measured by measuring the quantitative values of information corresponding to the absolute value at various locations along the optical fiber 12 using the O-ring test while the measurer M wearing the measuring apparatus 10 on his or her head is facing the target object.

If the quantitative value of information of the target object for measurement is high, then in order to reduce the burden on the assistant who performs the O-ring tests, measurement can also be performed based on one of the following methods, which utilize the addibility of the polarities and values of quantitative value information. Namely, in those cases where the quantitative value of information has been increased through the use of a quantitative value information transmitting apparatus disclosed in the non-patent reference 2 or the patent reference 2, measurements can be conducted either with auxiliary objects with a known quantitative value of information attached to the optical fiber 12, or with an auxiliary object with a known quantitative value of information provided in iterative contact with the optical fiber 12.

### [Example 1]

Before starting experimentation using the measuring apparatus 10, an auxiliary object used during measurement is prepared. A method of preparing this auxiliary object is described with reference to concrete examples. A first example involves the preparation of an auxiliary object with a quantitative value of information of (+1). Using the quantitative value information transmitting apparatus described in the patent reference 2, a 20 cc ampule of 5% glucose (with a known quantitative value of information of (+5)) and a 20 cc ampule of 50% glucose (with a known quantitative value of information of (-4)) were arranged in series in front of the energy transmission direction of the transmitting apparatus, as transmission source objects, and a booklet (a bundle of small pieces of paper) was positioned at a position further forward than the transmission source objects, as a transmission target object. The combined quantitative value information of the 20 cc ampule of 5% glucose and the 20 cc ampule of 50% glucose (resulting in a quantitative value of information of (+1)) was transmitted to the booklet using the quantitative value information transmitting apparatus. This transmission causes the pieces of paper in the booklet to each adopt a quantitative value of information of (+1). The auxiliary object in this first example shall be known, for now, as auxiliary object X1.

A second example involves the preparation of an auxiliary object with a quantitative value of information of (-1). In the same manner as the first example, a 20 cc ampule of 5% glucose (with a known quantitative value of information of (+5)) and 5 cc of an Oxyfull, hydrogen peroxide solution (with a known quantitative value of information of (-6)) were arranged in series in front of the energy transmission direction of the transmitting apparatus, as transmission source objects. A booklet was positioned at a position further forward than the transmission source objects as the transmission target object, and the combined quantitative value information of the 20 cc ampule of 5% glucose and the 5 cc of Oxyfull, hydrogen peroxide solution (resulting in a quantitative value of information of (-1)) was transmitted to the booklet. This transmission causes the pieces of paper in the booklet to each adopt a quantitative value of information of (-1). The auxiliary object in this second example shall be known, for now, as auxiliary object X2. The auxiliary object X1 with the quantitative value of information of (+1) and the auxiliary object X2 with the quantitative value of information of (-1) are both meaningful as units. By changing the type and quantity of transmission source objects, it is possible to prepare an auxiliarY object with any desired quantitative value of information.

A third example involves the preparation of an auxiliary object with a quantitative value of information of (+20). A 20 cc ampule of Minophagen C (with a known quantitative value of information of (+20), see the non-patent reference 2) was placed in front of the energy transmission direction of the transmitting apparatus, as a transmission source object, a booklet was placed at a position further forward than the transmission source object as the transmission target object, and the quantitative value information of the 20 cc ampule of Minophagen C was transmitted to the booklet. This transmission causes the pieces of paper in the booklet to each adopt a quantitative value of information of (+20). The auxiliary object in this third example shall be known, for now, as auxiliary object X3. In a fourth example, by transmitting the quantitative value information of a capsule of EPA/DHA cs(with a known quantitative value of information of (+80), see the non-patent reference 2) to a booklet in a similar manner, each of the pieces of paper in the booklet adopts a quantitative value of information of (+80). The auxiliary object in this fourth example shall be known, for now, as auxiliary object X4.

Using the measuring apparatus 10, and with a sample of p53 Activator (fusion peptide 46, a cancer-inhibiting gene made by BIOMOL Research Laboratories) placed at a distance of 50 cm as the target object for measurement, the quantitative value of information was measured. The quantitative value information of the p53 Activator (fusion peptide 46) that is generated in the pineal body part P when the measurer M wearing the measuring apparatus 10 on his or her head faces the object, flows through the information transmission circuit from the pineal body part P to the left side touqiaoyin L, the optical fiber end section 12a, the optical fiber end section 12b, the right side touqiaoyin R, and back to the pineal body part P, as described previously. Since the quantitative value of information of the p53 Activator (fusion peptide 46) used as the target object for measurement in this case was unknown, the quantitative value of information was measured using auxiliary objects.

First, the O-ring test was used to confirm that the position at the center C of the optical fiber showed a value of <0>. Next, to determine the polarity of the field comprising the optical fiber 12 and the target object for measurement, the O-ring test was performed in this field. In the O-ring test performed in this field, because the O-ring stayed closed, the polarity of the field was determined to be (+), that is, the polarity of the quantitative value of information of the target object for measurement was determined to be (+), and because the first attempt of the O-ring test felt quite strong, it was assumed that the quantitative value of information of the target object for measurement was quite high. As such, auxiliary objects with high quantitative values of information were used from the outset. A piece of paper (deemed auxiliary object X5) with a quantitative value of information of (+946), obtained by transmitting the quantitative value information of a single vial of an extract including deer antler, denshichi, schisandra, and deer uterus, which are high-grade Japanese and Chinese medicines, was used, together with the auxiliary objects X3 and X4 described above. Because the polarity of the field was (+), the auxiliary objects with a (+) value were attached between the center C of the optical fiber 12 and the optical fiber end section 12b, that is, in the portion where the polarity of the information flowing through the optical fiber 12 was (+).

Next, the O-ring test was performed with one piece of the auxiliary object X5 paper attached to the right side of the optical fiber 12. With one piece of the auxiliary object X5 paper attached the O-ring still did not open, that is, the result was (+), and therefore more pieces of the auxiliary object X5 paper were added, one by one, and the O-ring test was performed after the attachment of each additional piece. Because the O-ring was opened when the O-ring test was performed with four pieces of the paper attached, that is, the (+) quantitative value of information of the attached auxiliary objects was too high, one of the pieces of auxiliary object X5 paper was removed to lower the (+) value toward <0>, and pieces of paper of another auxiliary object X4 with a lower quantitative value of information were added, one by one, with the O-ring test once again performed after the attachment of each piece. This time, the O-ring was opened when the O-ring test was performed with six pieces of the auxiliary object X4 paper attached, and consequently, one of the pieces of the auxiliary object X4 paper was removed, a piece of the auxiliary object X3 paper with an even lower quantitative value of information was added, and the O-ring test was performed again. Fig. 4 is a diagram showing the state where three pieces of the auxiliary object X5 paper, 5 pieces of the auxiliary object X4 paper and 1 piece of the auxiliary object X3 paper are attached to the right side of the optical fiber 12. At this point in time, the value at the center position C of the optical fiber was <0>, meaning the total quantitative value of information of the auxiliary objects X5, X4, and X3, namely (+946) x 3 + (+80) x 5 + (+20) = (+3358), represents the quantitative value of information of the p53 Activator (fusion peptide 46) target object.

### [Example 2]

In the example 1, the quantitative value of information of an object with an unknown quantitative value of information was measured by employing a method in which quantitative value information was sent from an object with a known quantitative value of information into pieces of paper, using a quantitative value information transmitting apparatus, and the pieces of paper that had received this quantitative value of information were then attached to the optical fiber 12 as auxiliary objects. The example 2 described below proves that it is also possible to measure quantitative values of information using an auxiliary object iterative contact method, by measuring the quantitative value of information of an object with a known quantitative value of information, using an object with a known quantitative value of information as the auxiliary object, and employing an iterative contact method in which this auxiliary object is placed in iterative contact with the optical fiber 12.

It is possible to prepare several different types of objects with different quantitative values of information as the auxiliary objects, in a similar manner to the example 1, but because in the present example a 20 cc ampule of Minophagen C (with a known quantitative value of information of (+20)) was used as the target object for measurement, a substance consisting of a 20 mg ATP injection solution ampule bound with ATPase 1ut1V, which has a quantitative value of information of (+1) (see pp. 43 to 44 of the non-patent reference 2), was used as the auxiliary object. A method for measuring the quantitative value of information of the target object for measurement using this 20 mg ATP injection solution ampule bound with ATPase 1 ut 1V (for the sake of convenience, hereafter this is abbreviated as ATP) as the auxiliary object is described below.

By performing the O-ring test under conditions where the measurer M is wearing the measuring apparatus 10 on his or her head and facing the object, it was determined that the polarity of the field, that is, the polarity of the quantitative value of information of the target object for measurement, was (+). Because the polarity of the field was (+), the auxiliary object ATP, which has a quantitative value of information of (+1), was placed in iterative contact with the portion of the optical fiber 12 between the center C and the optical fiber end section 12b, that is, the portion where the polarity of the information flowing through the optical fiber 12 is (+). This method of iterative contact involves holding the ATP manually, and repeatedly bringing the ATP and the optical fiber 12 into contact by tapping the ATP against the optical fiber 12. Each time the ATP comes into contact with the optical fiber 12, quantitative value information of (+1) flows into the optical fiber 12. Repeating this contact causes the quantitative value of information to accumulate, and the absolute value of the quantitative value of information flowing in the optical fiber 12 will have a value equivalent to the number of times contact has occurred.

The absolute value of the quantitative value of information of the target object for measurement was determined by performing the O-ring test at the center position of the optical fiber 12 between iterations of this iterative contact, and counting the number of times iterative contact of the ATP had occurred before the value evaluated by the O-ring test was <0>. In this example, the value determined by the O-ring test was <0> when the number of repetitions of iterative contact had reached 20, and because the polarity was (+), the quantitative value of information was therefore identified as being (+20). This value (+20) is the same as the known quantitative value of information of the 20 cc ampule of Minophagen C, and these results prove that it is also possible to measure quantitative values of information using an auxiliary object iterative contact method.

Although the description above focuses mainly on the use of medicine-related objects as the object within the quantitative value information system, the object need not be related to medicine, and the quantitative value of information can be measured for any object which affects the person who represents the subject directly or indirectly in his or her life, including food, drinking water, lifestyle products, and toxic substances in the environment.

## Claims

1. A measuring apparatus for measuring quantitative values of information in a quantitative value information system which evaluates a biological tolerance relationship between a pineal body part of a brain of a person, who represents a subject, and an object, the apparatus comprising:
a hat body capable of being worn on a head of the person;
a cord attached to the hat body, with end sections that point to right and left touqiaoyin, respectively, which are meridian points in a rear temporal region, and which forms a circular arc shape to a rear of the head in its entirety; and
a metal antenna coil which is attached to the hat body and points to a forehead of the person.

2. The measuring apparatus for measuring quantitative values of information according to claim 1, wherein
either an optical fiber or a conductive wire is used as the cord.

3. The measuring apparatus for measuring quantitative values of information according to claim 1, wherein
metal auxiliary coils are attached to the respective ends of the cord.

4. The measuring apparatus for measuring quantitative values of information according to claim 2, wherein
metal auxiliary coils are attached to the respective ends of the cord.

5. A measuring method for measuring quantitative values of information in a quantitative value information system which evaluates a biological tolerance relationship between a pineal body part of a brain of a person, who represents a subject, and an object, the method comprising the steps of:
allowing a measurer to place the measuring apparatus according to any one of claims 1 to 4 on a head of the measurer so that the end sections of the cord point to the respective touqiaoyin points in the rear temporal region;
using an O-ring test to determine a quantitative value of information for information flowing through the cord of the measuring apparatus when the measurer faces the object for measurement; and
determining, when a value for the O-ring test at a center of the cord in a lengthwise direction is zero <0>, the quantitative value of information of the object for measurement from a value of the quantitative value of information flowing through the cord at a position other than the center of the cord.

6. The measuring method for measuring quantitative values of information according to claim 5, comprising the steps of:
attaching an auxiliary object with a known quantitative value of information to the cord at a position other than the center position, or providing an auxiliary object with a known quantitative value of information in iterative contact with the cord at a position other than the center position, and
determining the quantitative value of information of the object for measurement from the determined value of the quantitative value of information flowing through the cord, except at the center of the cord, when the value of the O-ring test at the center position of the cord is zero <0>.
